# FASCICULE DE BREVET EUROPEEN

(11) **EP 2 666 071 B1**
(45) Date de publication et mention de la délivrance du brevet: **02.12.2020**
(21) Numéro de dépôt: 12704859.3
(22) Date de dépôt: 10.01.2012
(51) Int. Cl.: G06F 3/01, A61B 3/113, F16M 13/00, G02B 27/00, G03B 15/16, G06K 11/00, G06K 9/52, G05B 19/00, G06F 3/033, H04N 5/235, H04N 13/00

(54) **PROCEDE DE DETERMINATION DE LA DIRECTION DU REGARD ET DISPOSITIF POUR SA MISE EN OEUVRE**
VERFAHREN ZUR FESTSTELLUNG DER BLICKRICHTUNG UND VORRICHTUNG DAFÜR
METHOD FOR DETERMINING GAZE DIRECTION AND DEVICE FOR SAME

(30) Priorité: 19.01.2011 FR 1150405
(43) Date de publication de la demande: 27.11.2013
(73) Titulaire: Matchic Labs, 75011 Paris (FR)
(72) Inventeur: GUITTEAUD, Eric Clément, F-75020 Paris (FR); BASCOULERGUE, Pierre Youri, F-75020 Paris (FR)
(74) Mandataire: Fédit-Loriot
(86) Numéro de dépôt international: PCT/FR2012/050065
(87) Numéro de publication internationale: WO 2012/098325

(56) Documents cités:
- FR-A1- 2 937 428
- FR-A1- 2 937 428
- GB-A- 2 467 301
- GB-A- 2 467 301
- US-A- 4 836 670
- US-A- 4 836 670
- US-A1- 2002 013 573
- US-A1- 2002 013 573
- US-A1- 2005 175 218
- US-A1- 2005 175 218
- US-A1- 2010 025 444
- US-A1- 2010 025 444
- US-A1- 2010 053 555
- US-A1- 2010 053 555

## Description

La présente invention concerne le domaine des technologies liées à la mesure et au suivi de la direction du regard (« eyetracking » selon la terminologie anglo-saxonne) et concerne plus particulièrement, selon un premier de ses aspects, un procédé de suivi du regard d'un utilisateur sur une surface d'affichage observée par l'utilisateur, constituée notamment par l'écran d'un terminal mobile tel qu'un téléphone portable ou une tablette tactile, permettant de déterminer, en mobilité, à quels endroits de la surface d'affichage observée se pose le regard de l'utilisateur.

A titre d'exemple non exclusif d'application de l'invention on peut notamment citer l'analyse des interactions du regard avec une interface utilisateur, menée dans le cadre d'études comportementales visant par exemple à évaluer des zones d'intérêt au sein de cette interface ou à adapter l'ergonomie de cette interface.

Dans l'art antérieur, on distingue deux grandes familles de systèmes de suivi du regard:
- les systèmes non intrusifs et non portables ;
- les systèmes intrusifs et portables.

Tous ces systèmes utilisent un principe technique de base consistant à évaluer la direction du regard à partir de la position relative du reflet cornéen d'une source lumineuse et de la pupille.

Le principe consiste à disposer en tant que source lumineuse une DEL (diode électroluminescente) émettant un rayonnement dans les longueurs d'ondes de l'infrarouge juste à côté de l'objectif d'une caméra vidéo destinée à acquérir des images de l'œil. Cette DEL infrarouge émet une lumière vers l'œil dans la même direction que l'axe de l'objectif de la caméra vidéo. Cette lumière se réfléchit sur la cornée de l'oeil : un point lumineux, nommé « point de Purkinje », apparaît alors sur l'image de la cornée de l'œil capturée par la caméra vidéo.

Le système de suivi non-intrusif est un système qui permet d'enregistrer les mouvements de l'œil et donc de suivre le regard lorsque le sujet regarde par exemple un écran d'ordinateur.

Ce type de système est généralement composé d'une caméra très haute définition disposée à une soixantaine de centimètres de la tête de l'utilisateur dans l'axe du regard du sujet.

Cette caméra étant de haute définition, il est possible alors de distinguer le visage de l'utilisateur auquel elle fait face. Puis, par traitement d'images et reconnaissance de formes, il est possible d'identifier l'emplacement des yeux et de faire un agrandissement dans l'image pour déterminer avec précision la position de la pupille par rapport au point de Purkinje. Ce système n'interagit pas avec l'utilisateur. Ce dernier n'a pas à porter de lunette spéciale ou de casque.

Toutefois ce système a pour principal inconvénient de reposer sur l'utilisation d'une caméra haute définition dont le coût dépasse de plus de 100 fois le prix d'une caméra à résolution standard. De plus, lors de l'analyse du regard, l'utilisateur ne peut pas bouger la tête au-delà d'une limite tridimensionnelle définie par l'angle de vue de la caméra qui filme l'œil.

En outre, l'écran que regarde l'utilisateur doit obligatoirement être fixe, ce qui constitue une limitation importante, empêchant l'adaptation de ce type de système aux écrans d'affichage de terminaux mobiles tels que des téléphones portables ou tablettes tactiles par exemples, qui, par nature, sont utilisés en mouvement.

Les systèmes de suivi de regard intrusifs permettent d'enregistrer ce suivi du regard lorsque l'utilisateur regarde une scène globale comme le cockpit d'un avion par exemple ou encore le tableau de bord d'une voiture.

Ce type de système est généralement composé d'une première caméra vidéo disposée à une dizaine de centimètres de la tête de l'utilisateur dans l'axe de regard de l'utilisateur. Cette caméra vidéo est proche de la tête, elle doit donc être fixée à une paire de lunette spécifique ou à un casque.

Cette caméra vidéo filmant directement la zone de l'œil, par traitement d'image il est possible de reconnaître avec précision la position de la pupille par rapport au point de Purkinje.

Ainsi, ce système confère à l'utilisateur une grande liberté de mouvement. L'utilisateur peut ainsi se déplacer tout en laissant à la caméra la possibilité d'analyser la direction de son regard. De plus, un tel système permet l'utilisation de tous types de caméras ayant un coût réduit comparées par exemple aux caméras à haute définition.

Ce type de système comprend également une seconde caméra vidéo, dite caméra de scène, pour filmer la scène observée par l'utilisateur. Les deux flux vidéo issus respectivement de la première caméra vidéo et de la seconde caméra vidéo sont traités pour produire un flux vidéo unique représentant la scène et incluant un pointeur permettant de suivre les endroits de la scène où l'utilisateur pose son regard à chaque instant. Un tel système est bien connu de l'homme du métier, notamment par l'exemple qu'en donne le document US2010/0053555, qui décrit l'ensemble des caractéristiques contenues dans le préambule de la revendication 1.

Cependant, un inconvénient de ce système réside dans le fait que la première caméra vidéo servant à déterminer la position de l'œil et la seconde caméra vidéo permettant de filmer la scène observée par l'utilisateur sont dans un même repère, qui est lié au repère de la tête de l'utilisateur. Aussi, ce système n'est-il pas bien adapté au suivi du regard sur la surface d'un objet mobile, et, plus particulièrement, sur l'écran d'affichage d'un terminal mobile, tel qu'un téléphone portable, dans un contexte de mobilité. En effet, lorsque la caméra de scène enregistre une scène où l'utilisateur est en train de regarder, en mobilité, l'écran d'un téléphone portable par exemple, l'objet en question regardé et filmé devient mobile dans le flux d'images vidéo résultant de l'enregistrement de la scène par la caméra de scène. Il est alors particulièrement difficile de pouvoir déterminer automatiquement ce que l'utilisateur, en mobilité, est en train de regarder.

Par exemple, si l'utilisateur regarde continuellement une zone d'intérêt sur l'écran du téléphone portable dans un contexte de mobilité, le point de regard de l'utilisateur ne bouge pas en fonction de ladite zone d'intérêt mais, sur le flux d'images vidéo résultant de l'enregistrement de la scène par la caméra de scène, il se déplace en fonction de la position dans l'image de la zone d'intérêt regardée, elle-même mobile dans le flux vidéo résultant.

Dès lors, dans ce contexte de mobilité, il n'est pas possible de déterminer aisément et surtout de manière automatique, que l'utilisateur a fixé continuellement la zone d'intérêt en question, sauf, ce qui n'est pas souhaitable, à doter le système de puissants moyens de traitement d'images et reconnaissance de formes, aptes à reconnaître avec précision dans le flux d'images vidéo la zone d'intérêt en question, pour en déduire que le point de regard sur l'image a bien suivi cette zone d'intérêt, mobile dans l'image.

On connaît également dans l'état de la technique, le document FR 2 937 428 A1, qui concerne un système de modulation d'une source lumineuse de type diode électroluminescente infrarouge, permettant de synchroniser des périodes d'allumage de cette source en réponse à des signaux temporels correspondant à des champs de trames vidéo entrelacées, ces signaux étant détectés dans un signal vidéo d'une caméra vidéo. Ce système trouve une application à la localisation du point de Purkinje pour l'interaction homme machine par le suivi du regard de l'opérateur. Ce document décrit également un dispositif destiné à être porté sur la tête, ou en forme de lunettes ou similaire à un casque, supportant la caméra vidéo et source lumineuse devant l'œil de l'utilisateur.

Le document US 2005/175218 A1 concerne également, pour une même application, un système de synchronisation de périodes d'éclairage d'une source lumineuse de type diode électroluminescente infrarouge, en réponse à des signaux temporels correspondant à des trames vidéo, lesdits signaux temporels provenant de l'horloge de synchronisation d'une caméra.

Le document US 4 836 670 A décrit quant à lui un système de détection de mouvement oculaire qui utilise une diode électroluminescente infrarouge montée sur la caméra pour illuminer le visage. Le signal vidéo analogique provenant de la caméra est numérisé et une carte d'acquisition d'images acquiert et stocke une image de sorte que les lignes d'une paire de champs pairs et impairs successifs soient correctement fusionnées dans la mémoire de trame.

Le document US2002/013573 propose la modulation et/ou la synchronisation d'une lumière de capture oculaire (eye tracking) afin de filtrer la composante de lumière ambiante dans le signal d'image capturé; cela au moyen d'une source LED, et d'un appareil photo numérique rapide; le système peut être appliqué à un dispositif thérapeutique ou diagnostique.

Le document US2010/025444 divulgue un ensemble de support avec plaque de berceau pour terminal mobile, la plaque de berceau étant rotative pour permettre une orientation paysage ou portrait.

Enfin le document GB2467301 décrit un support pour terminal mobile ou ordinateur portable, dans lequel une source de lumière est montée sur un bras flexible qui s'étend depuis l'un des éléments du support.

Dans ce contexte, l'invention a pour but de remédier aux inconvénients de l'état de la technique et, en particulier, de s'affranchir des problèmes rencontrés par les systèmes connus de suivi du regard de type intrusif et portable pour pouvoir déterminer aisément et de manière automatique, dans un contexte de mobilité, la direction du regard par rapport à un objet regardé, notamment un téléphone portable.

Ce but est atteint par le procédé selon la revendication 1 et le système selon la revendication 6.

D'autres particularités et avantages de l'invention ressortiront à la lecture de la description faite ci-après d'un mode de réalisation particulier de l'invention, donné à titre indicatif mais non limitatif, en référence aux dessins annexés sur lesquels :
- la Figure 1 une vue d'ensemble du système de suivi de regard conforme à l'invention;
- la Figure 2 illustre schématiquement une vue de côté du dispositif conforme à l'invention pour supporter, en état de mobilité, l'objet observé par l'utilisateur ;
- la Figure 3 illustre une vue de dessus du dispositif de la figure 2;
- la Figure 4 illustre schématiquement une vue de profil de l'équipement portable destiné à être porté par l'utilisateur dans le cadre de la mise en œuvre du système de suivi de regard conforme à l'invention;
- la Figure 5 illustre une vue de dessus de l'équipement portable de la figure 4 ;
- la Figure 6 illustre une vue de dessus du dispositif de la figure 2 sur lequel une mire de calibration a été disposée, et
- la Figure 7 illustre une image de l'œil capturée par la caméra de l'équipement portable illustré aux figures 4 et 5 lorsque le système est en fonctionnement.

Le système qui sera maintenant décrit à titre d'exemple est destiné à déterminer la position instantanée de la direction du regard d'un sujet humain, ci-après l'utilisateur, relativement à une surface d'affichage observée d'un objet se présentant en particulier sous la forme d'un écran d'affichage d'un terminal mobile, tel qu'un téléphone portable ou une tablette tactile par exemple. On se place dans un contexte d'utilisation de l'objet par l'utilisateur en mobilité réelle. Dès lors, dans le système de l'invention, l'objet, qui est normalement tenu en main par l'utilisateur lors de son utilisation, est prévu pour être mobile par rapport à la tête de l'utilisateur.

Comme il a été expliqué précédemment, les systèmes connus de suivi de regard de type intrusif et portable ne sont pas adaptés à l'enregistrement de scène où l'utilisateur est en train de regarder un objet dans un contexte de mobilité réelle, puisque l'objet regardé et filmé devient mobile dans la vidéo résultante de l'enregistrement de la scène par la caméra de scène. Aussi, tout en se situant dans la famille des systèmes de suivi de regard de type intrusif et portable, le système de l'invention propose de capturer la scène observée par l'utilisateur, c'est-à-dire typiquement l'écran du téléphone portable, dans le repère du téléphone portable et de positionner dans ce même repère des sources lumineuses de référence destinées à se réfléchir dans l'œil de l'utilisateur, ces sources étant en outre disposées relativement au téléphone portable dans le même plan que celui formé par l'écran du téléphone portable.

Ainsi, l'image de la surface d'affichage observée (l'écran) se trouvera toujours au même endroit dans la vidéo résultante de la caméra de scène et les reflets des sources lumineuses de référence permettront de lier après calcul, comme on le verra par la suite, la position du regard découlant de la position de la pupille dans l'image de l'œil à la position du regard dans le repère de la scène observée.

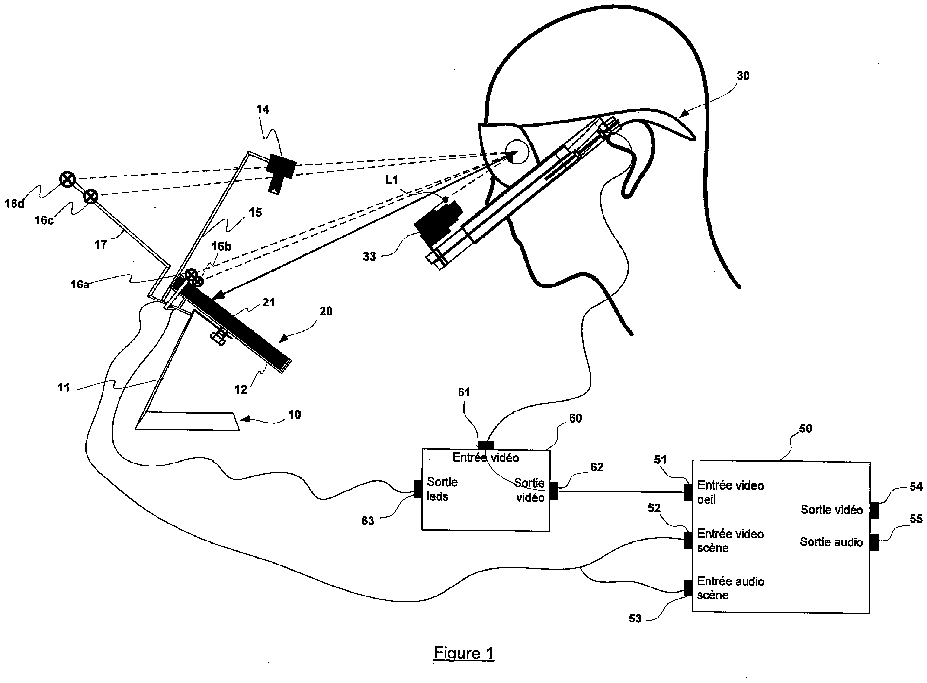

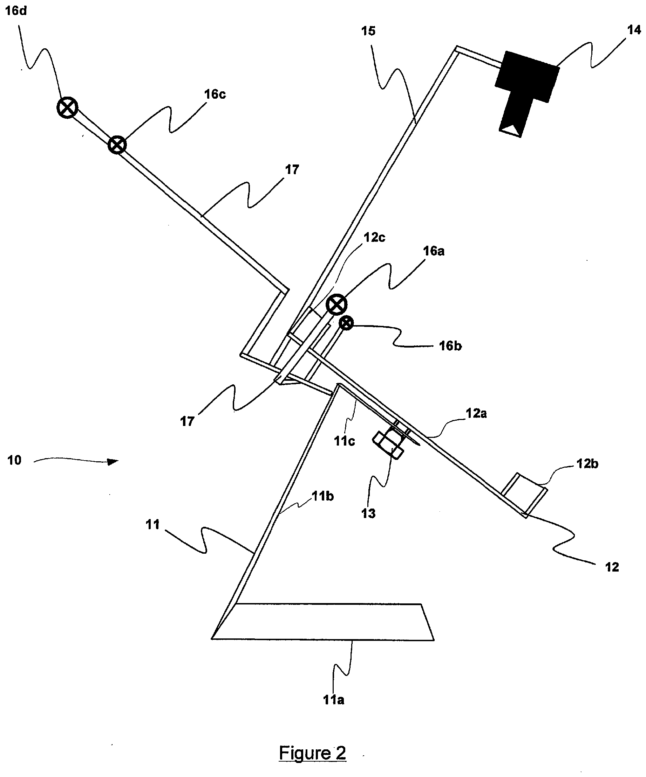

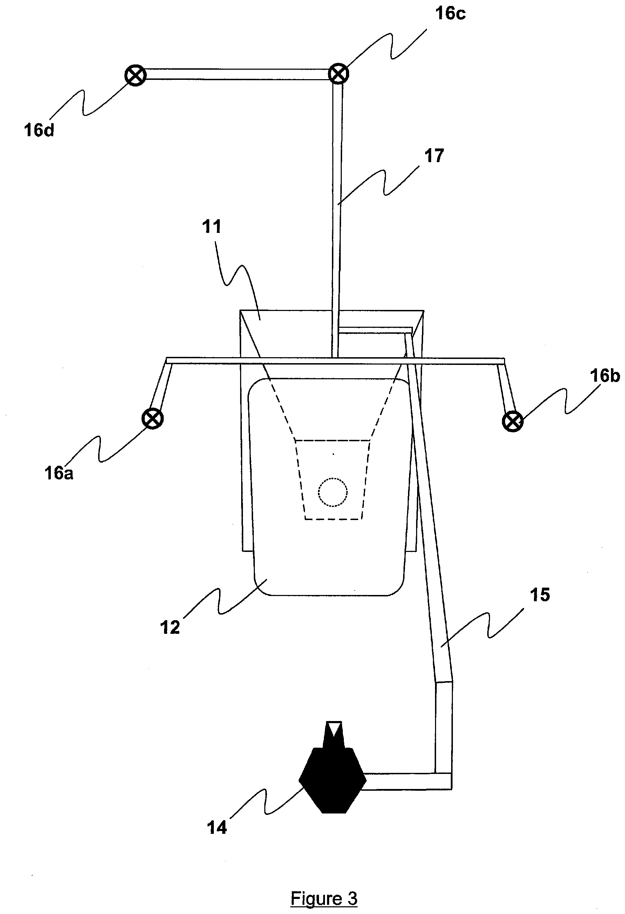

Pour ce faire, l'exemple de réalisation du système selon l'invention illustré à la figure 1 montre un dispositif 10 pour supporter, en état de mobilité, un terminal mobile 20 présentant une surface d'affichage 21 destinée à être regardée par l'utilisateur. Le terminal mobile est par exemple constitué par un téléphone portable et la surface d'affichage est constituée par l'écran du téléphone portable. Le dispositif 10, illustré plus précisément aux figures 2 et 3, comprend un élément support 11 destiné d'une part, à servir de socle pour le dispositif et d'autre part, à servir d'organe de préhension manuelle du dispositif. Il se présente sous la forme d'une pièce massive conformée en une première partie 11a formant un appui plan, une seconde partie 11b s'entendant depuis l'appui plan et formant une poignée permettant à l'utilisateur de tenir et manœuvrer l'ensemble, et une troisième partie 11c, s'étendant de manière inclinée depuis une extrémité de la partie 11b formant poignée et sur laquelle est monté un plateau 12. Le plateau 12 comporte une surface plane 12a munie par exemple de butées inférieure et supérieure 12b et 12c délimitant ainsi un logement destiné à recevoir le téléphone portable en position d'utilisation, i.e. dans une position dans laquelle l'écran d'affichage est apte à être observé par l'utilisateur, notamment lorsqu'il tient le dispositif en main. Le plateau 12 est prévu pour être monté mobile sur l'élément support 11 et, plus précisément, est monté à rotation sur la partie inclinée 11c de l'élément support. Le plateau 12 est par exemple muni d'une vis de serrage 13, permettant de verrouiller le plateau 12 sur la partie inclinée 11c de l'élément support 11 dans des positions définies. La vis 13 permet par exemple de verrouiller le plateau 12 dans deux positions sensiblement à angle droit entre elles, de sorte à définir respectivement une position dite portrait et une position dite paysage pour l'écran d'affichage du téléphone portable destiné à être reçu dans le logement.

Le dispositif 10 comprend en outre une caméra vidéo 14, dite caméra de scène, prévue pour capturer des images de l'écran du téléphone portable reçu dans le logement, dans le même référentiel que celui du téléphone portable. Pour ce faire, la caméra de scène 14 est soutenue au-dessus du plateau 12 formant logement pour le téléphone portable par une armature légère 15 équipant le dispositif, qui est liée à l'élément de support 11, de préférence dans une zone supérieure de la partir 11b formant poignée, de sorte à ne pas gêner la préhension de l'ensemble. Cette armature de soutien 15 conçue pour soutenir la caméra de scène 14 dans le référentiel du téléphone portable reçu dans le logement du dispositif 10 comprend par exemple un bras fixé par une première de ses extrémités à l'élément de support 11 et s'étendant depuis cette première extrémité vers une seconde extrémité positionnée au-dessus du plateau formant logement. Cette seconde extrémité du bras est munie de moyens de fixation de la caméra de scène 14, non représentés, permettant de maintenir la caméra de scène 14 dans la bonne orientation. Grâce à cet agencement, la caméra de scène 14 prévu pour filmer l'écran du téléphone portable reçu dans le logement du dispositif 10 est fixée par rapport au téléphone portable dans le référentiel du téléphone portable lui-même. Aussi, l'écran regardé et filmé demeurera fixe dans la vidéo résultante de la capture de l'écran par la caméra de scène 14, y compris dans un contexte de mobilité où, par exemple, l'utilisateur bouge la tête par rapport à l'écran regardé ou encore déplace le téléphone portable dans l'espace en même temps qu'il regarde l'écran.

Selon une variante de réalisation, le signal vidéo de l'écran observé par l'utilisateur, peut être capturé en utilisant directement la sortie vidéo du terminal mobile, dans les cas où le terminal mobile présente une telle sortie vidéo permettant classiquement d'afficher l'écran du terminal mobile sur un support d'affichage externe (écran de télévision ou d'ordinateur par exemple). Selon cette variante de réalisation, la présence d'une caméra de scène équipant le dispositif 10 comme expliqué plus haut n'est pas nécessaire.

De surcroît, afin de pouvoir déterminer en mobilité à quels endroits de l'écran se pose le regard de l'utilisateur, le dispositif comprend une pluralité de sources lumineuses de référence 16a, 16b, 16c, 16d, qui sont donc positionnées dans le même référentiel que celui du téléphone portable reçu dans le logement et qui sont prévues pour émettre une lumière vers l'utilisateur destinée à se réfléchir sur l'œil de l'utilisateur. Les sources lumineuses de référence 16a, 16b, 16c, 16d sont de préférence constituées de quatre diodes électroluminescentes (DELs) émettant un rayonnement dans les longueurs d'ondes de l'infrarouge. Les quatre DELs infrarouges sont plus précisément disposées dans l'environnement du logement, selon un réseau en deux dimensions situé dans le même plan que celui formé par l'écran du téléphone portable reçu dans le logement et présentent en outre la caractéristique de ne pas être alignées trois par trois. Le plan formé par les quatre DELs infrarouges 16a à 16d est disposé parallèlement au plan formé par la surface plane 12a du plateau 12 formant logement pour le téléphone portable, et est situé légèrement au-dessus de celui-ci, d'une hauteur tenant compte de l'épaisseur de l'objet reçu dans le logement, de sorte que les quatre DELs soient positionnées dans le même plan que celui formé par l'écran de l'objet.

Les quatre DELs infrarouges de référence sont soutenues autour du plateau 12 formant logement pour recevoir le téléphone portable, par une armature légère 17 équipant le dispositif et qui est liée à l'élément de support 11, de préférence dans la même zone de l'élément support 11 à partir de laquelle se déploie l'armature légère de soutien 15 de la caméra de scène 14. Cette armature de soutien 17 des quatre DELs infrarouges est par exemple constituée de quatre bras, présentant chacun une extrémité libre sur laquelle est fixée une DEL infrarouge de sorte que cette dernière émette une lumière vers l'œil de l'utilisateur, l'autre extrémité de chacun des bras de l'armature de soutien 17 étant fixé à l'élément support 11 du dispositif. Les bras de l'armature de soutien sont agencés les uns aux autres de sorte à satisfaire aux contraintes géométriques de positionnement des DELs infrarouges telles qu'énoncées ci-dessus.

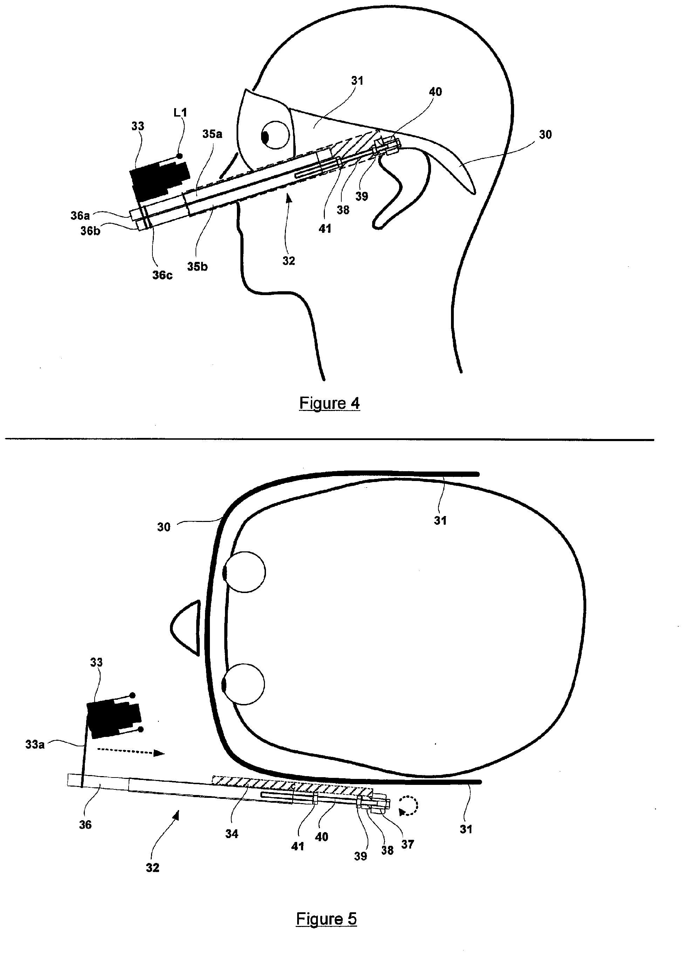

L'exemple de réalisation du système selon l'invention illustré à la figure 1 montre également un équipement 30 de type intrusif et portable pour l'identification de la position de l'œil par rapport à un point lumineux réfléchi sur la cornée nommé « point de Purkinje ». Cet équipement, qui est destiné à être attaché à l'utilisateur est décrit plus précisément aux figures 4 et 5. Il comprend un support 30 adapté à être ajusté à la tête de l'utilisateur. Le support 30 est ici réalisé sous forme d'une monture de lunettes démunie de verres correcteurs, et comprenant deux branches 31 dont l'une au moins est suffisamment large au moins dans sa zone avant pour recevoir latéralement le un bras 32 qui sert à solidariser une caméra vidéo miniature 33 à la monture 30. Comme on le verra plus en détail par la suite, la longueur du bras est réglable par un mécanisme à vis sans fin 36.

Le bras 32 est constitué d'une plaque de base allongée 34, fixée par exemple par collage à la branche 31 de monture et prolongeant celle-ci vers l'avant. Deux tubes parallèles 35a, 35b sont fixés par tout moyen, par exemple par des cordons de colle, à la plaque de support 34 et servent de corps de guidage à deux tiges coulissantes 36a, 36b réalisées par exemple sous forme de tubes minces. Les extrémités distales des deux tiges 36a, 36b, dépassent des tubes 35a, 35b et sont assujetties l'une à l'autre par des moyens 36c, par exemple par des colliers de serrage avec une entretoise interposée, ou encore par des rivets traversant l'ensemble des deux tubes. Toujours à l'extrémité distale, les mêmes moyens 36c, ou d'autres moyens 33a, peuvent être utilisés pour fixer la caméra vidéo 33 aux tiges 36a, 36b, dans une orientation fixée, sensiblement dans l'axe du regard de l'utilisateur. Du côté proximal, les extrémités des tiges 36a, 36b dépassent également des tubes extérieurs 35a, 35b. L'ensemble des deux tiges 36a, 36b peut donc coulisser sans tourner dans les tubes 35a, 35b. On pourrait aussi prévoir une seule tige ayant une section non cylindrique pour garantir l'absence de rotation, mais la présente réalisation a l'avantage de permettre un coulissement très doux et de ne nécessiter que des éléments extrêmement courants et peu coûteux du commerce.

À son extrémité proximale, la plaque 34 reçoit en équerre un guide axial 38 comportant un passage axial lisse aligné avec l'axe du tube 35b et de la tige 36b. Ce passage accueille la tige filetée 40 d'une vis sans fin. Un écrou 39 de butée est enfilé et bloqué, par exemple par collage, sur la tige filetée 40, au voisinage de la pièce de guidage axial 38, tandis qu'une molette de réglage 37 est collée à l'extrémité proximale de la vis, dont le mouvement axial est ainsi limité par la coopération en butée des éléments 37, 38 et 39, tandis que sa rotation libre est possible en actionnant la molette de réglage. La tige filetée 40 engrène dans un écrou 41 fixé, par exemple par collage, à l'extrémité proximale de la tige 10b.

Le fonctionnement de l'équipement est le suivant. La monture de lunettes est ajustée sur la tête de l'utilisateur, éventuellement après un préréglage comme mentionné plus haut. Le réglage fin de la netteté des images capturées par la caméra 33 est effectué par l'utilisateur à l'aide de la molette 37:
- Tourner la molette 37 dans un premier sens prédéfini fait se rapprocher l'élément 41 de l'élément 38. Or, les éléments 41, 36c, 35a, 35b, et 33 sont tous solidaires, donc la caméra se rapproche de l'œil de l'utilisateur.
- Tourner la molette dans le sens inverse au premier sens prédéfini fait s'éloigner l'élément 41 de l'élément 38. Or, les éléments 41, 36c, 35a, 35b, et 33 sont tous solidaires, donc la caméra s'éloigne de l'œil de l'utilisateur.

Grâce à cet agencement, le rapprochement ou l'éloignement de la caméra 33 de la position de l'œil de l'utilisateur, et donc le réglage de la netteté, s'effectue facilement et d'une seule main, à l'aide d'une simple rotation de la molette 37 disposée à l'extrémité proximale du bras 32, à proximité de l'oreille, comme indiqué dans la figure 4, c'est-à-dire à un endroit fixe par rapport à la tête du sujet où la manœuvre de la molette ne risque pas de déplacer malencontreusement l'ensemble du dispositif, qui n'a donc pas besoin d'être tenu.

L'équipement comprend en outre une première source lumineuse L1 dirigée vers l'œil et située à proximité de l'objectif de la caméra vidéo. Elle est par exemple fixée directement sur le corps de la caméra 33. Cette première source lumineuse est de préférence constituée d'une DEL émettant un rayonnement dans les longueurs d'ondes de l'infrarouge, nommée par la suite DEL de « Purkinje ». Au besoin, on pourra ajouter une seconde source lumineuse L2, de type DEL infrarouge, dirigée vers l'œil et disposée à proximité de l'objectif de la caméra vidéo, de la même manière que pour la source L1, pour le cas d'utilisation du système dans des endroits particulièrement sombres.

L'exemple de réalisation du système selon l'invention illustré à la figure 1 montre également une unité de traitement 50 comprenant une première interface d'entrée vidéo 51 pour recevoir le signal vidéo capturé par la caméra vidéo 33 de l'équipement portable ajusté sur l'utilisateur et une seconde interface d'entrée vidéo 52 pour recevoir le signal vidéo capturé par la caméra de scène 14, ainsi qu'une interface d'entrée audio 53, permettant de recevoir le signal audio issu du microphone équipant la caméra de scène. L'unité de traitement comprend également au moins une interface de sortie vidéo 54 et une interface de sortie audio 55.

Ainsi, lorsque la caméra de scène 14 capture des images de l'écran du téléphone portable disposé sur le dispositif support 10 et observé par l'utilisateur, les images de l'écran sont transmises à l'unité de traitement 50 sous forme d'un signal vidéo à partir d'une liaison connectée à l'interface d'entrée vidéo 52 de l'unité de traitement et sont stockées dans des moyens de mémorisation appropriées de l'unité de traitement. Selon la variante de réalisation décrite plus haut sans caméra de scène, l'interface d'entrée vidéo 52 de l'unité de traitement 50 est prévue pour être connectée sur la sortie vidéo du terminal mobile reçu dans le logement du dispositif 10, tandis que l'interface d'entrée audio 53 peut être connectée à un microphone d'ambiance. Dans cette variante, l'enregistrement vidéo de l'écran observé par l'utilisateur est donc fourni directement par le terminal mobile lui-même, via la sortie vidéo du terminal mobile.

Egalement, lorsque la caméra vidéo 33 de l'équipement portable ajusté sur la tête de l'utilisateur capture des images de l'œil de l'utilisateur, les images de l'œil de l'utilisateur sont transmises à l'unité de traitement 50 sous forme d'un signal vidéo à partir d'une liaison connectée à l'interface d'entrée vidéo 51 de l'unité de traitement et sont stockées dans des moyens de mémorisation appropriées de l'unité de traitement.

Toutefois, dans un mode de réalisation préféré, le système comprend encore un module 60 de synchronisation de l'allumage de la DEL de « Purkinje » et des DELs de référence avec la capture du signal vidéo par la caméra vidéo 33 de l'équipement portable. Ce module est intercalé entre la caméra vidéo 33 et l'unité de traitement 50. Il comprend une interface d'entrée vidéo 61 pour recevoir le signal vidéo acquis par la caméra 33 de l'équipement portable et une interface de sortie vidéo 62. Il comprend également des moyens 63 d'alimentation de DEL aptes à contrôler de manière synchrone l'allumage et l'extinction de la DEL de « Purkinje » et des DELs de référence.

Ainsi, selon ce mode de réalisation préférée, lorsque la caméra vidéo 33 de l'équipement portable ajusté sur la tête de l'utilisateur capture des images de l'œil de l'utilisateur, les images de l'œil sont transmises au module de synchronisation 60 sous forme d'un signal vidéo à partir d'une liaison connectée à l'interface d'entrée vidéo 61 du module de synchronisation, et sont stockées dans des moyens de mémorisation appropriées du module de synchronisation pour traitement. Parallèlement au traitement du signal vidéo par le module de synchronisation, ce signal vidéo est retransmis à l'unité de traitement 50 à partir d'une liaison connectant l'interface de sortie vidéo 62 du module de synchronisation à l'interface d'entrée vidéo 51 de l'unité de traitement 50, sans modification et tel qu'il a été reçu sur l'interface d'entrée vidéo 61.

Le module de synchronisation 60 a pour rôle de détecter un signal de synchronisation contenu dans le signal vidéo reçu et de commander l'allumage de la DEL de « Purkinje » et des DELs de référence en fonction de ce signal de synchronisation, de sorte à synchroniser l'allumage de ces DELs avec la capture d'une image. Plus particulièrement, les quatre DELs de référence 16a à 16d et la DEL de « Purkinje » L1 sont commandées par le module de synchronisation pour clignoter d'une manière synchrone au rythme d'une image sur deux par rapport aux images de l'œil capturée par la caméra 33 de l'équipement portable. On entend par synchrone le fait que lors de l'enregistrement du signal vidéo par la caméra 33 de l'équipement portable, les DELs seront par exemple allumées pendant l'enregistrement des images impaires tandis qu'elles seront éteintes pendant l'enregistrement des images paires. En effet, le fait que les DELs soient allumées une image sur deux seulement permettra de mieux discriminer la position effective des reflets lumineux des DELs dans l'image résultante de l'œil.

Plus précisément, selon un exemple de réalisation, le module de synchronisation utilise des composants électroniques afin d'extraire du signal vidéo de la caméra 33 une première information relative au top trame paire et impaire de synchronisation de ce signal vidéo et de traiter cette information à partir de la détection d'une deuxième information extraite du signal vidéo relative au top image paire et impaire restituées par le signal vidéo. Rappelons qu'une image est composée de deux trames (une trame impaire et une trame paire) et qu'un flux vidéo est composé d'une succession d'images numérotées de 1 à l'infini dont le groupe des images impaires (numérotées 1, 3, 5, ...) et le groupe des images paires (numérotées 2, 4, 6, ...). Donc, quand deux images se succèdent l'une est impaire et l'autre est forcément paire.

Ce traitement du signal vidéo issu de la caméra 33 peut par exemple correspondre à un calcul effectué par le module de synchronisation sur la base de ces informations permettant d'identifier la trame impaire de l'image impaire dans le signal vidéo, afin d'allumer les DELs au moment où est identifiée la trame impaire de l'image impaire, ce qui équivaut à éclairer la scène de la trame impaire que la caméra est en train de capturer. De la sorte, la durée d'éclairage des DELs est rigoureusement égale au temps d'une seule trame (soit le temps d'une demi-image). L'allumage des DELs s'effectuant sur la durée d'une trame impaire seulement et démarrant au début d'une image complète, on est certain que l'éclairage se fera obligatoirement sur une image complète et ne pourra pas se chevaucher sur l'image suivante. A cette fin, le module de synchronisation met en œuvre des moyens de traitement aptes à extraire une première information relative au top trame paire et impaire de synchronisation de ce signal vidéo, à traiter cette première information de sorte à obtenir une deuxième information relative au top image paire et impaire restituées par le signal vidéo et à effectuer une opération de type ET logique entre les trames impaires et les images impaires obtenues de sorte à déterminer la commande de déclenchement/d'extinction des DELs à envoyer au module d'alimentation des DELs.

Il est à noter, en variante, qu'on peut utiliser une caméra numérique permettant de récupérer directement le signal de synchronisation des images paires et impaires. En effet de telles caméras numériques mettent avantageusement à disposition le top de synchronisation image sans qu'il soit nécessaire de l'extraire du signal vidéo.

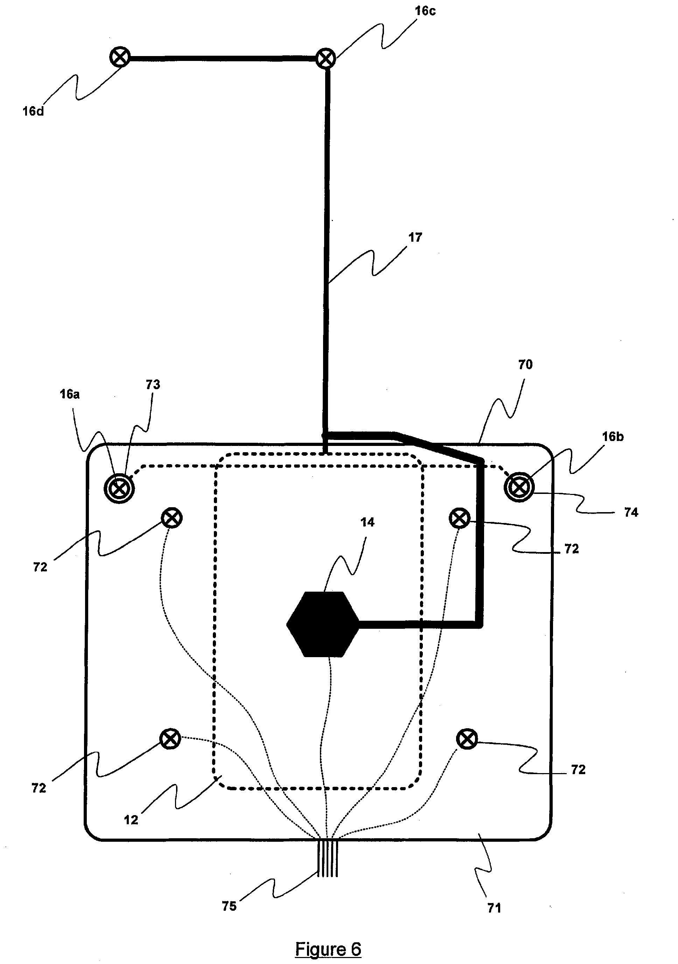

Le fonctionnement du système tel qu'il vient d'être décrit nécessite une calibration, d'une part des paramètres intrinsèques de l'œil de l'utilisateur (chaque utilisateur ayant a priori un œil différent) et des paramètres de positionnement du plateau 12 dans le champ de la caméra de scène 14. Pour ce faire, comme illustrée à la figure 6, on utilise une mire 70 positionnée sur le plateau 12 du dispositif 10. La mire 70 se présente sous la forme d'une plaque 71 sur laquelle sont fixées selon une forme géométrique prédéfinie cinq DELs de calibration 72 émettant une lumière vers l'œil de l'utilisateur, sur lequel on a ajusté au préalable l'équipement portable 30. Par exemple, quatre DEL sont disposés sur la plaque 71 de sorte à former un carré et la cinquième DEL est disposé au centre du carré, cette dernière étant masquée par la caméra 14 sur la vue de dessus de la figure 6. Les DELs de calibration 72 utilisées sont des DELs de couleur visible à l'œil nu lorsqu'elles sont allumées, de préférence de couleur rouge.

Selon l'exemple de la figure 6, la plaque 71 est munie de deux trous 73, 74, à travers lesquels on fait passer les deux DELs de référence 16a, 16b, lors du positionnement de la plaque 71 sur le plateau 12, de sorte que pendant l'opération de calibration, la plaque 71 est parfaitement centrée par rapport au plateau 12 et reste solidaire de ce dernier.

L'allumage des cinq DELs de calibration 72 est réalisé individuellement à l'aide d'une commande électronique spécifique transmise par l'intermédiaire d'un câble de liaison 75 reliant les DELs de calibration de la plaque à une unité de commande d'alimentation des DELs de calibration non représentée.

L'opération de calibration consiste plus précisément à fixer les cinq DELs de calibration clignotantes les unes après les autres. A partir de l'image de l'œil capturée pendant l'opération de calibration, on mémorise les différentes positions de l'œil et des points de réflexion cornéenne pour chacune des DELs de calibration clignotantes fixée par l'œil de l'utilisateur. Suite à la mémorisation de ces différentes positions, on procède au calcul des paramètres intrinsèques de l'œil. Dès lors, il est possible de déterminer une bijection entre la direction du regard déterminée par une corrélation de la position du point de Purkinje et du centre de la pupille dans l'image de l'œil et le plan formé par les quatre DELs de référence 16a à 16d.

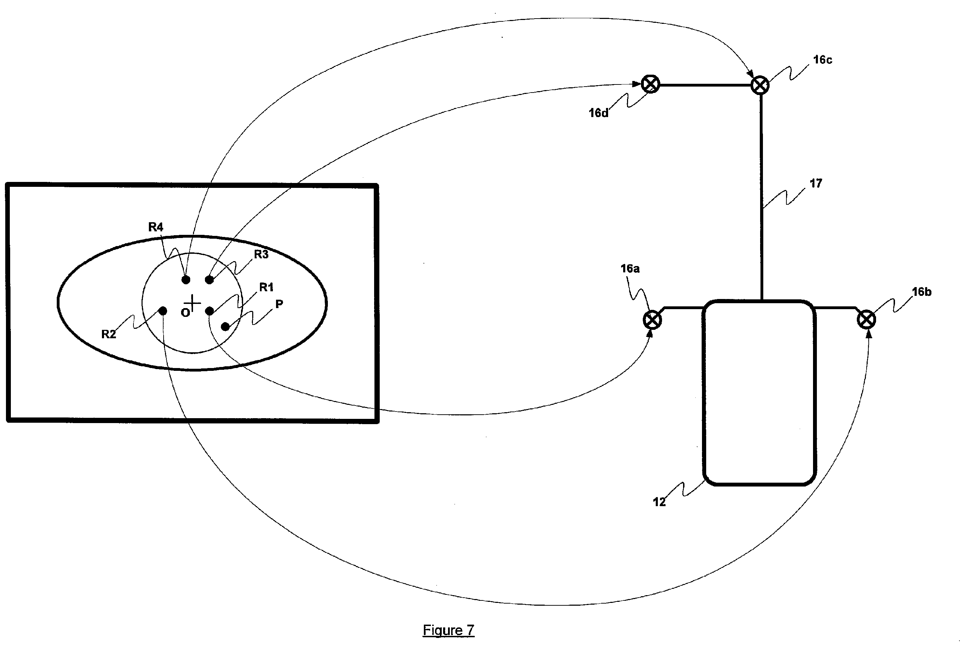

En fonctionnement, comme il a été expliqué précédemment, les quatre DELs de référence 16a à 16d du dispositif 10 pour supporter, en état de mobilité, l'objet regardé par l'utilisateur et la DEL de « Purkinje » L1 de l'équipement portable 30 ajusté sur la tête de l'utilisateur clignotent d'une manière synchrone au rythme d'une image sur deux par rapport aux images capturées par la caméra vidéo 33 de l'équipement portable 30. Lorsque les DELs 16a à 16d et L1 sont allumées, elles se reflètent sur l'œil de l'utilisateur et permettent donc d'avoir un point fixe dans l'image de l'œil capturée grâce à la réflexion cornéenne. Ainsi, pour deux images successives capturées, une des deux images contient l'image des reflets cornéens des DELs allumées et l'autre image non. Une méthode de soustraction entre les deux images avec un traitement par seuillage mis en œuvre par l'unité de traitement 50 recevant le signal vidéo issu de la caméra 33 permet d'obtenir une image résultante telle qu'illustrée à la figure 7, qui contient uniquement l'image des reflets cornéens des DELs. A partir d'algorithmes particuliers, l'unité de traitement est à même de déterminer la position du centre de la pupille et celle du centre des reflets cornéens dans l'image de l'œil. Partant, la position du reflet cornéen P de la DEL de « Purkinje » sur l'œil issue de la réflexion de la DEL de « Purkinje » sur l'œil et la position du centre de la pupille O permettent de déterminer, dans le repère de l'œil, la direction du regard, c'est-à-dire l'angle de regard de l'œil par rapport à la scène observée. Dès lors, après calcul, la position dans l'image des reflets cornéens R1, R2, R3 et R4 respectivement des quatre DELs de référence 16a, 16b, 16c et 16d et la direction du regard ainsi mesurée vont permettre de déterminer précisément la position du regard sur la surface observée de l'objet disposé sur le plateau 12 du dispositif support 10.

En effet, la détermination de la position du centre des reflets cornéens des quatre DELs de référence 16a à 16d dans l'image de l'œil permet de déterminer, dans le repère de l'œil, la position du regard par rapport à celle de la surface observée sur la surface de l'œil. Il est alors possible, par changement de repère, d'en déduire la position du regard dans le référentiel de la caméra de scène 14 qui définit un plan formé par les quatre DELs de référence 16a à 16d correspondant au plan de la surface observée. La position du regard ainsi déterminée dans le plan de la surface observée est utilisée pour afficher dans la vidéo de scène un pointeur représentatif de cette position du regard. Pour ce faire l'unité de traitement opère au préalable une synchronisation des signaux vidéo issus respectivement de la caméra de scène et de la caméra vidéo de l'équipement portable afin d'ajuster les instants significatifs correspondants de ces deux signaux vidéo.

Grâce aux moyens de l'invention, en particulier le dispositif pour supporter l'objet en état de mobilité, l'image de la surface observée se trouvera toujours au même endroit dans la vidéo de scène et les DELs de référence permettent de déterminer, après calculs, la position du regard de l'utilisateur par rapport à l'image de la surface observée. De ce fait, il est beaucoup plus aisé de créer des cartes de zones d'intérêt d'une interface utilisateur d'un téléphone portable par exemple puisque sur une période d'utilisation donnée de ce téléphone portable dans un contexte de mobilité réelle, l'interface utilisateur telle que regardée et filmée sera toujours au même endroit dans la vidéo de scène et seul le point de regard sera mobile par rapport à l'image de l'interface utilisateur.

## Revendications

1. Procédé de suivi du regard d'un utilisateur sur une surface d'affichage (21) d'un terminal mobile (20) observée par l'utilisateur consistant à déterminer la position du regard de l'utilisateur sur la surface d'affichage observée, dans lequel on fournit une première caméra vidéo (33) située dans l'axe du regard de l'utilisateur et une première source lumineuse (L1) dirigée vers l'œil et située à proximité de l'objectif de la première caméra vidéo (33), pour recueillir un premier signal vidéo comportant au moins l'image de la pupille et celle du reflet cornéen de la première source lumineuse, on recueille un second signal vidéo comportant des images de la surface d'affichage (21), on analyse le premier signal vidéo pour mesurer dans le repère de l'œil la direction du regard en fonction de la position du centre de la pupille (O) par rapport au centre du reflet cornéen (P) de la première source lumineuse (L1) et on affiche dans le second signal vidéo un pointeur représentatif de la position du regard sur la surface d'affichage observée en fonction de la direction du regard mesurée dans le repère de l'œil,
le procédé étant **caractérisé en ce qu'**on capture le second signal vidéo dans un référentiel distinct du référentiel de la première caméra vidéo (33), lié à celui de la surface d'affichage observée, on positionne dans le référentiel de capture du second signal vidéo une pluralité de sources lumineuses de référence (16a, 16b, 16c, 16d) dirigées vers l'œil et disposées selon un réseau en deux dimensions coplanaire avec la surface d'affichage observée, de sorte que le premier signal vidéo recueilli par la première caméra vidéo (33) comporte en outre l'image des reflets cornéens (R1, R2, R3, R4) des sources lumineuses de référence sur l'œil, on analyse le premier signal vidéo pour fournir la position du centre des reflets cornéens des sources lumineuses de référence sur l'œil, on détermine la position du regard dans le plan de la surface d'affichage observée à partir de la position du centre des reflets cornéens des sources lumineuses de référence sur l'œil et de la direction du regard mesurée dans le repère de l'œil, on détermine dans le repère de l'œil, à partir de la position du centre des reflets cornéens des sources lumineuses de référence sur l'œil, la position du regard par rapport à la position de la surface observée sur l'œil et on utilise une relation de conversion entre un système de coordonnées lié au repère de l'œil et un système de coordonnées lié au plan formé par la pluralité de sources lumineuses de référence correspondant au plan de la surface d'affichage observée pour déterminer la position du regard dans le plan de la surface d'affichage observée en fonction de la position du regard déterminée dans le repère de l'œil.

2. Procédé selon la revendication 1, **caractérisé en ce qu'**on effectue au préalable une opération de calibration du positionnement de la surface observée dans le référentiel de capture du second signal vidéo en fonction de l'œil de l'utilisateur, consistant à calculer la position du centre de la pupille sur chacun des points de fixation (72) d'une mire (70) prédéfinie positionnée dans le plan de la surface observée permettant de définir des points de correspondance entre le plan de la surface observée et l'image de l'œil.

3. Procédé selon l'une quelconque des revendications précédentes, **caractérisé en ce qu'**on fournit une seconde source lumineuse (L2) dirigée vers l'œil à proximité de l'objectif de la première caméra vidéo (33) destinée à favoriser l'éclairement de l'œil.

4. Procédé selon l'une quelconque des revendications précédentes, **caractérisé en ce qu'**on fournit une seconde caméra vidéo (14) positionnée dans le référentiel de la surface d'affichage observée pour capturer le second signal vidéo.

5. Procédé selon l'une quelconque des revendications 1 à 3, **caractérisé en ce qu'**on capture le second signal vidéo en utilisant une sortie vidéo du terminal mobile.

6. Système de suivi du regard d'un utilisateur selon le procédé de l'une quelconque des revendications 1 à 5 sur une surface d'affichage d'un terminal mobile (20) en état de mobilité, le système comprenant :
un dispositif (10) pour supporter ledit terminal mobile comprenant un élément de support (11) formant un organe de préhension manuelle du dispositif et un plateau (12) monté mobile par rapport à l'élément de support (11), le plateau (12) constituant un logement destiné à recevoir le terminal mobile en position d'utilisation dans laquelle la surface d'affichage (21) est apte à être observée par un utilisateur, le dispositif comprenant une pluralité de sources lumineuses de référence (16a, 16b, 16c, 16d) liées à l'élément de support (11), aptes à émettre une lumière vers l'œil de l'utilisateur et disposées dans l'environnement du logement selon un réseau en deux dimensions coplanaire avec la surface d'affichage (21) du terminal mobile destiné à être reçu dans le logement,
un équipement portable attaché à l'utilisateur comprenant un support (30) adapté à être ajusté à la tête de l'utilisateur et un bras (32) monté sur le support (30) et portant à son extrémité distale une première caméra vidéo (33), dans l'axe de regard de l'utilisateur, et une première source lumineuse (L1) dirigée vers l'œil et située à proximité de l'objectif de la première caméra vidéo (33), et
une unité de traitement (50) comprenant une première interface d'entrée vidéo (51) apte à recevoir un premier signal vidéo capturé par la première caméra vidéo (33) de l'équipement portable comportant l'image de la pupille, celle du reflet cornéen de la première source lumineuse (L1) dirigée vers l'œil et celle des reflets cornéens correspondant à chacune des sources lumineuses de référence (16a, 16b, 16c, 16d) équipant le dispositif (10) pour supporter le terminal mobile, une seconde interface d'entrée vidéo (52) apte à recevoir un second signal vidéo comportant l'image de la surface d'affichage observée par l'utilisateur, et un module de calcul apte à mesurer, à partir du premier signal vidéo, la direction du regard dans le repère de l'œil en fonction de la position du centre de la pupille par rapport au centre du reflet cornéen de la première source lumineuse et à déterminer la position du regard dans le plan de la surface d'affichage observée, à partir de la position du centre des reflets cornéens des sources lumineuses de référence dans le premier signal vidéo et de la direction du regard mesurée dans le repère de l'œil, ledit module de calcul étant apte à déterminer dans le repère de l'œil, à partir de la position du centre des reflets cornéens des sources lumineuses de référence sur l'œil, la position du regard par rapport à la position de la surface observée sur l'œil et à utiliser une relation de conversion entre un système de coordonnées lié au repère de l'œil et un système de coordonnées lié au plan formé par la pluralité de sources lumineuses de référence correspondant au plan de la surface d'affichage observée pour déterminer la position du regard dans le plan de la surface d'affichage observée en fonction de la position du regard déterminée dans le repère de l'œil.

7. Système selon la revendication 6, **caractérisé en ce que** le dispositif (10) pour supporter le terminal mobile comprend une seconde caméra vidéo (14) pour fournir le second signal vidéo, la seconde caméra vidéo (14) étant liée à l'élément de support (11) du dispositif (10) pour supporter le terminal mobile et étant apte à être orientée vers le logement destiné à recevoir le terminal mobile pour capturer des images de la surface d'affichage observée, et **en ce que** la seconde interface d'entrée vidéo (52) de l'unité de traitement (50) est connectée à une sortie vidéo de la seconde caméra vidéo (14).

8. Système selon la revendication 6, **caractérisé en ce que** la seconde interface d'entrée vidéo (52) de l'unité de traitement (50) est connectée à une sortie vidéo du terminal mobile apte à fournir le second signal vidéo.

9. Système selon l'une quelconque des revendications 6 à 8, **caractérisé en ce que** l'unité de traitement comprend une interface de sortie vidéo (54) apte à fournir le second signal vidéo dans lequel les images de la surface d'affichage observée affichent un pointeur représentatif de la position du regard dans le plan de la surface observée déterminée par le module de calcul.

10. Système selon l'une quelconque des revendications 6 à 9, **caractérisé en ce qu'**il comprend un module de synchronisation (60) de l'allumage de la première source lumineuse (L1) et des sources lumineuses de référence (16a, 16b, 16c, 16d) avec la capture du premier signal vidéo par la première caméra vidéo (33) de l'équipement portable, ledit module de synchronisation comprenant une interface d'entrée vidéo (61) apte à recevoir le premier signal vidéo de la première caméra vidéo (33) de l'équipement portable, des moyens d'alimentation (63) aptes à contrôler de manière synchrone l'allumage et l'extinction de la première source lumineuse et des sources lumineuses de référence et des moyens de traitement du signal vidéo reçu agencés avec lesdits moyens d'alimentation de sorte à commander l'allumage de la première source lumineuse et des sources lumineuses de référence au rythme d'une image sur deux par rapport aux images capturées par la première caméra vidéo de l'équipement portable.

11. Système selon l'une quelconque des revendications 6 à 10, **caractérisé en ce que** le support (30) de l'équipement portable est une monture de lunettes à deux branches, le bras (32) étant fixé sur une des branches de la monture.

12. Système selon l'une quelconque des revendications 6 à 11, **caractérisé en ce que** le bras (32) monté sur le support (30) de l'équipement portable porte une seconde source lumineuse (L2) dirigée vers l'œil à proximité de l'objectif de la première caméra vidéo (33) et destinée à favoriser l'éclairement de l'œil.

13. Système selon l'une quelconque des revendications 6 à 12, **caractérisé en ce que** la pluralité de sources lumineuses de référence (16a, 16b, 16c, 16d) est constituée de quatre diodes électroluminescentes non alignées lorsqu'elles sont considérées trois par trois.

14. Système selon l'une quelconque des revendications 6 à 13, **caractérisé en ce que** le plateau (12) du dispositif pour supporter ledit terminal mobile est monté à rotation sur l'élément support (11) entre au moins deux positions sensiblement à angle droit entre elles, de sorte à définir respectivement une position dite portrait et une position dite paysage pour la surface d'affichage du terminal mobile destiné à être reçu dans le logement, et **en ce qu'**il comprend des moyens de verrouillage (13) du plateau dans chacune de ces positions respectives.

## Patentansprüche

1. Verfahren zur Verfolgung des Blicks eines Benutzers auf eine Anzeigeoberfläche (21) eines mobilen Endgeräts (20), die von dem Benutzer betrachtet wird, das darin besteht, die Position des Blicks des Benutzers auf der betrachteten Anzeigeoberfläche festzustellen, wobei eine erste Videokamera (33), die sich in der Achse des Blicks des Benutzers befindet, und eine erste Lichtquelle (L1) bereitgestellt werden, die hin zum Auge gerichtet ist und sich in der Nähe des Objektivs der ersten Videokamera (33) befindet, derart dass ein erstes Videosignal erfasst wird, das mindestens das Bild der Pupille und das des Reflexes der ersten Lichtquelle auf der Hornhaut umfasst, ein zweites Videosignal erfasst wird, das Bilder der Anzeigeoberfläche (21) umfasst, das erste Videosignal derart analysiert wird, dass im Bezugspunkt des Auges die Blickrichtung in Abhängigkeit von der Position des Mittelpunkts der Pupille (O) in Bezug auf den Mittelpunkt des Reflexes der ersten Lichtquelle (L1) auf der Hornhaut (P) gemessen wird und in dem zweiten Videosignal ein Zeiger, der für die Position des Blicks auf der betrachteten Anzeigeoberfläche repräsentativ ist, in Abhängigkeit von der in dem Bezugspunkt des Auges gemessenen Blickrichtung angezeigt wird, wobei das Verfahren **dadurch gekennzeichnet ist, dass** das zweite Videosignal in einem Bezugssystem erfasst wird, das sich von dem Bezugssystem der ersten Videokamera (33) unterscheidet und das mit demjenigen der betrachteten Anzeigeoberfläche verbunden ist, in dem Erfassungsbezugssystem des zweiten Videosignals eine Vielzahl von Bezugslichtquellen (16a, 16b, 16c, 16d) platziert werden, die hin zu dem Auge gerichtet sind und gemäß einem zweidimensionalen Netz angeordnet sind, das koplanar mit der betrachteten Anzeigeoberfläche ist, derart dass das erste Videosignal, das von der ersten Videokamera (33) erfasst wird, ferner das Bild der Reflexe (R1, R2, R3, R4) der Bezugslichtquellen auf dem Auge auf der Hornhaut umfasst, das erste Videosignal so analysiert wird, dass die Position des Mittelpunktes der Reflexe der Bezugslichtquellen auf dem Auge auf der Hornhaut bereitgestellt wird, die Position des Blicks in der Ebene der betrachteten Anzeigeoberfläche ausgehend von der Position des Mittelpunkts der Reflexe der Bezugslichtquellen auf dem Auge auf der Hornhaut und der in dem Bezugspunkt des Auges gemessenen Blickrichtung festgestellt wird, in dem Bezugspunkt des Auges ausgehend von der Position des Mittelpunkts der Reflexe der Bezugslichtquellen auf dem Auge auf der Hornhaut die Position des Blicks in Bezug auf die Position der betrachteten Oberfläche auf dem Auge festgestellt wird und eine Umsetzungsbeziehung zwischen einem Koordinatensystem verwendet wird, das mit dem Bezugspunkt des Auges verbunden ist, und einem Koordinatensystem, das mit der Ebene verbunden ist, die von der Vielzahl von Bezugslichtquellen gebildet ist, die der Ebene der betrachteten Anzeigeoberfläche entsprechen, sodass die Position des Blicks in der Ebene der betrachteten Anzeigeoberfläche in Abhängigkeit von der in dem Bezugspunkt des Auges bestimmten Position des Blicks festgestellt wird.

2. Verfahren nach Anspruch 1, **dadurch gekennzeichnet, dass** im Voraus ein Vorgang zur Kalibrierung der Positionierung der betrachteten Oberfläche in dem Bezugssystem der Erfassung des zweiten Videosignals in Abhängigkeit von dem Auge des Benutzers ausgeführt wird, der darin besteht, die Position des Mittelpunkts der Pupille auf jedem der Fixierungspunkte (72) einer vordefinierten Zielvorrichtung (70), die in der Ebene der betrachteten Oberfläche positioniert ist, zu berechnen, wodurch das Definieren von Entsprechungspunkten zwischen der Ebene der betrachteten Oberfläche und dem Bild des Auges ermöglicht wird.

3. Verfahren nach einem der vorhergehenden Ansprüche, **dadurch gekennzeichnet, dass** eine zweite Lichtquelle (L2), die hin zu dem Auge gerichtet ist, in der Nähe des Objektivs der ersten Videokamera (33) bereitgestellt wird, die dazu bestimmt ist, die Beleuchtung des Auges zu begünstigen.

4. Verfahren nach einem der vorhergehenden Ansprüche, **dadurch gekennzeichnet, dass** eine zweite Videokamera (14) bereitgestellt wird, die so in dem Bezugssystem der betrachteten Anzeigeoberfläche positioniert ist, dass das zweite Videosignal erfasst wird.

5. Verfahren nach einem der Ansprüche 1 bis 3, **dadurch gekennzeichnet, dass** das zweite Videosignal unter Verwendung eines Videoausgangs des mobilen Endgeräts erfasst wird.

6. System zur Verfolgung des Blicks eines Benutzers nach dem Verfahren nach einem der Ansprüche 1 bis 5 auf einer Anzeigeoberfläche eines mobilen Endgeräts (20) im Mobilitätszustand, wobei das System umfasst:
eine Vorrichtung (10) zum Stützen des mobilen Endgeräts, die ein Stützelement (11), das ein Organ zum Greifen der Vorrichtung mit der Hand bildet, und eine Platte (12) umfasst, die in Bezug auf das Stützelement (11) beweglich gelagert ist, wobei die Platte (12) eine Aufnahme bildet, die dazu bestimmt ist, das mobile Endgerät in der Verwendungsposition aufzunehmen, in der die Anzeigeoberfläche (21) geeignet ist, von einem Benutzer betrachtet zu werden, wobei die Vorrichtung eine Vielzahl von Bezugslichtquellen (16a, 16b, 16c, 16d) umfasst, die mit dem Stützelement (11) verbunden sind, geeignet sind, ein Licht hin zu dem Auge des Benutzers zu emittieren, und in der Umgebung der Aufnahme gemäß einem zweidimensionalen Netz angeordnet sind, das koplanar mit der Anzeigeoberfläche (21) des mobilen Endgeräts ist, das dazu bestimmt ist, in der Aufnahme aufgenommen zu werden,
eine tragbare Ausrüstung, die an dem Benutzer befestigt ist und eine Stütze (30), die so angepasst ist, dass sie auf den Kopf des Benutzers eingestellt ist, und einen Arm (32) umfasst, der an der Stütze (30) montiert ist und an seinem distalen Ende eine erste Videokamera (33) in der Achse des Blicks des Benutzers und eine erste Lichtquelle (L1) trägt, die hin zu dem Auge gerichtet ist und sich in der Nähe des Objektivs der ersten Videokamera (33) befindet, und eine Verarbeitungseinheit (50), die eine erste Videoeingangsschnittstelle (51), die geeignet ist, ein erstes Videosignal zu empfangen, das von der ersten Videokamera (33) der tragbaren Ausrüstung erfasst wird, das das Bild der Pupille, das des Reflexes der ersten Lichtquelle (L1), die in Richtung des Auges gerichtet ist, auf der Hornhaut, und das der Reflexe auf der Hornhaut umfasst, die jeder der Bezugslichtquellen (16a, 16b, 16c, 16d) entsprechen, mit denen die Vorrichtung (10) zum Stützen des mobilen Endgeräts ausgerüstet ist, eine zweite Videoeingangsschnittstelle (52), die geeignet ist, ein zweites Videosignal zu empfangen, das das Bild der von dem Benutzer betrachteten Anzeigeoberfläche umfasst, und ein Rechenmodul umfasst, das geeignet ist, ausgehend von dem ersten Videosignal die Richtung des Blicks in dem Bezugspunkt des Auges in Abhängigkeit von der Position des Mittelpunkts der Pupille in Bezug auf den Mittelpunkt des Reflexes der ersten Lichtquelle auf der Hornhaut zu messen und die Position des Blicks in der Ebene der betrachteten Anzeigeoberfläche ausgehend von der Position des Mittelpunkts der Reflexe der Bezugslichtquellen auf der Hornhaut in dem ersten Videosignal und der Richtung der in dem Bezugspunkt des Auges gemessenen Blickrichtung festzustellen, wobei das Rechenmodul geeignet ist, in dem Bezugspunkt des Auges ausgehend von der Position des Mittelpunkts der Reflexe der Bezugslichtquellen auf dem Auge auf der Hornhaut die Position des Blicks in Bezug auf die Position der betrachteten Oberfläche auf dem Auge festzustellen und eine Umsetzungsbeziehung zwischen einem Koordinatensystem, das mit dem Bezugspunkt des Auges verbunden ist, und einem Koordinatensystem, das mit der Ebene verbunden ist, die von der Vielzahl von Bezugslichtquellen gebildet wird, die der Ebene der betrachteten Anzeigeoberfläche entspricht, zu verwenden, um die Position des Blicks in der Ebene der betrachteten Anzeigeoberfläche in Abhängigkeit von der Position des in dem Bezugspunkt des Auges bestimmten Blicks festzustellen.

7. System nach Anspruch 6, **dadurch gekennzeichnet dass** die Vorrichtung (10) zum Stützen des mobilen Endgeräts eine zweite Videokamera (14) zum Bereitstellen des zweiten Videosignals umfasst, wobei die zweite Videokamera (14) so mit dem Stützelement (11) der Vorrichtung (10) verbunden ist, dass das mobile Endgerät gestützt wird, und geeignet ist, hin zu der Aufnahme ausgerichtet zu sein, die dazu bestimmt ist, das mobile Endgerät aufzunehmen, um Bilder der betrachteten Anzeigeoberfläche aufzunehmen, und dadurch, dass die zweite Videoeingangsschnittstelle (52) der Verarbeitungseinheit (50) mit einem Videoausgang der zweiten Videokamera (14) verbunden ist.

8. System nach Anspruch 6, **dadurch gekennzeichnet, dass** die zweite Videoeingangsschnittstelle (52) der Verarbeitungseinheit (50) mit einem Videoausgang des mobilen Endgeräts verbunden ist, der geeignet ist, das zweite Videosignal bereitzustellen.

9. System nach einem der Ansprüche 6 bis 8, **dadurch gekennzeichnet, dass** die Verarbeitungseinheit eine Videoausgangsschnittstelle (54) umfasst, die geeignet ist, das zweite Videosignal bereitzustellen, in dem die Bilder der betrachteten Anzeigeoberfläche einen Zeiger anzeigen, der für die Position des Blicks in der Ebene der betrachteten Oberfläche repräsentativ ist, die von dem Rechenmodul bestimmt wird.

10. System nach einem der Ansprüche 6 bis 9, **dadurch gekennzeichnet, dass** es ein Modul (60) zur Synchronisation des Einschaltens der ersten Lichtquelle (L1) und der Bezugslichtquellen (16a, 16b, 16c, 16d) mit der Erfassung des ersten Videosignals durch die erste Videokamera (33) der tragbaren Ausrüstung umfasst, wobei das Synchronisationsmodul eine Videoeingangsschnittstelle (61), die geeignet ist, das erste Videosignal der ersten Videokamera (33) der tragbaren Ausrüstung aufzunehmen, Versorgungsmittel (63), die geeignet sind, das Einschalten und das Ausschalten der ersten Lichtquelle und der Bezugslichtquellen auf synchrone Weise zu steuern, und Mittel zur Verarbeitung des empfangenen Videosignals umfasst, die derart mit den Versorgungsmitteln angeordnet sind, dass das Einschalten der ersten Lichtquelle und der Bezugslichtquellen im Rhythmus jedes zweiten Bildes in Bezug auf die von der ersten Videokamera der tragbaren Ausrüstung aufgenommenen Bilder geregelt wird.

11. System nach einem der Ansprüche 6 bis 10, **dadurch gekennzeichnet, dass** die Stütze (30) der tragbaren Ausrüstung ein Brillengestell mit zwei Bügeln ist, wobei der Arm (32) an einem der Bügel des Gestells fixiert ist.

12. System nach einem der Ansprüche 6 bis 11, **dadurch gekennzeichnet, dass** der Arm (32), der an der Stütze (30) der tragbaren Ausrüstung montiert ist, eine zweite Lichtquelle (L2), die hin zu dem Auge gerichtet ist, in der Nähe des Objektivs der ersten Videokamera (33) trägt, und dazu bestimmt ist, die Beleuchtung des Auges zu begünstigen.

13. System nach einem der Ansprüche 6 bis 12, **dadurch gekennzeichnet, dass** die Vielzahl der Bezugslichtquellen (16a, 16b, 16c, 16d) aus vier Leuchtdioden besteht, die nicht ausgerichtet sind, wenn sie in Dreiergruppen betrachtet werden.

14. System nach einem der Ansprüche 6 bis 13, **dadurch gekennzeichnet, dass** die Platte (12) der Vorrichtung zum Stützen des mobilen Endgeräts auf dem Stützelement (11) zwischen mindestens zwei Positionen drehbar gelagert ist, die im Wesentlichen rechtwinklig zueinander sind, derart dass jeweils eine sogenannte Hochformatposition und eine sogenannte Querformatposition für die Anzeigeoberfläche des mobilen Endgeräts definiert wird, das dazu bestimmt ist, in der Aufnahme aufgenommen zu werden, und dadurch, dass es Mittel (13) zur Verriegelung der Platte in jeder dieser jeweiligen Positionen umfasst.

## Claims

1. A method for tracking the user's gaze on a displaying surface (21) of a mobile terminal (20) viewed by the user, consisting in determining the position of the user's gaze on the viewed displaying surface, wherein a first video camera (33) is provided along the axis of the user's gaze and a first light source (L1) directed towards the eye and located near the lens of the first video camera (33), in order to collect a first video signal including at least the pupil image and that of the corneal reflection of the first light source, a second video signal including images of the displaying surface (21) is collected, the first video signal is analyzed to measure within the eye reference mark, the direction of the gaze according to the position of the pupil center (O) relatively to the corneal reflection center (P) of the first light source (L1), and, on the second video signal, a pointer representative of the gaze position on the viewed displaying surface according to the gaze direction measured in the eye reference mark is displayed, the method being **characterized in that** the second video signal is captured in a reference frame, separate from the reference frame of the first video camera (33), bound to that of the viewed displaying surface, within the capture reference frame of the second video signal, a plurality of reference light sources (16a, 16b, 16c, 16d) are positioned directed towards the eye and disposed according to a bi-dimensional network coplanar with the viewed displaying surface, so that the first video signal collected by the first video camera (33) further includes the image of the corneal reflections (R1, R2, R3, R4) of the reference light sources on the eye, the first video signal is analyzed to provide the position of the corneal reflections center of the reference light sources on the eye, the position of the gaze in the plan of the displaying surface viewed is determined from the position of the corneal reflections center of the reference light sources on the eye and the direction of the gaze measured in the eye reference mark, in the eye reference mark, from the position of the corneal reflections center of the reference light sources on the eye, the position of the gaze relatively to the position of the surface viewed on the eye is determined and a conversion relationship between a reference frame related to the eye reference mark and a reference frame related to the plan formed by the plurality of reference light sources corresponding to the plan of the viewed displaying surface is used to determine the position of the gaze in the plan of the viewed displaying surface according to the position of the gaze determined in the eye reference mark.

2. The method according to claim 1,
**characterized in that** a calibration operation of the viewed surface positioning within the capture reference frame of the second video signal according to the user's eye is performed beforehand, consisting to calculate the position of the pupil center on each attaching point (72) of a preset test pattern (70) positioned within the plan of the viewed surface allowing to define correspondence points between the viewed surface plan and the eye image.

3. The method according to any of preceding claims, **characterized in that** a second light source (L2) is provided, being directed towards the eye near the lens of the first video camera (33) in order to improve the eye illumination.

4. The method according to any of preceding claims, **characterized in that** a second video camera (14) is provided, being positioned in the reference frame of the viewed displaying surface in order to capture the second video signal.

5. The method according to any of claims 1 to 3, **characterized in that** the second video signal is captured by using a video output of the mobile terminal.

6. A system for tracking the user's gaze according to the method according to any one of claims 1 to 5 on a displaying surface of a mobile terminal (20) in a mobility state, the system comprising:
a device (10) intended to support said mobile terminal comprising a support element (11) making a manual gripping member for the device and a plate (12) movably fitted relatively to the support element (11), the plate (12) constituting a housing intended to receive the mobile terminal in an using position in which the displaying surface (21) is ready to be viewed by a user, the device comprising a plurality of reference light sources (16a, 16b, 16c, 16d) related to the support element (11), ready to emit a light towards the user's eye and disposed proximately to the housing according to a bi-dimensional network coplanar with the displaying surface (21) of the mobile terminal intended to be received into the housing,
a portable equipment attached to the user comprising a support (30) adapted to be adjusted to the user's head and an arm (32) fitted to the support (30) and carrying at its distal end a first video camera (33), along the axis of user's gaze, and a first light source (LI) directed towards the eye and located near the lens of the first video camera (33), and
a processing unit (50) comprising a first video input interface (51) ready to receive a first video signal captured by the first video camera (33) of the portable equipment including the pupil image, that of the corneal reflection of the first light source (L1) directed towards the eye and that of the corneal reflections corresponding to each reference light source (16a, 16b, 16c, 16d) equipping the device (10) intended to support the mobile terminal, a second video input interface (52) ready to receive a second video signal including the image of the displaying surface viewed by the user, and a calculation module suited to measure, from the first video signal, the gaze direction in the eye reference mark according to the position of the pupil center relatively to the corneal reflection center of the first light source and to determine the position of the gaze within the plan of the viewed displaying surface, from the position of the corneal reflections center of the reference light sources in the first video signal and the direction of the gaze measured in the eye reference mark, said calculation module being ready to determine in the eye reference mark, from the position of the corneal reflections center of the reference light sources on the eye, the position of the gaze relatively to the position of the surface viewed on the eye and to use a conversion relationship between a reference frame related to the eye reference mark and a reference frame related to the plan formed by the plurality of reference light sources corresponding to the plan of the viewed displaying surface to determine the position of the gaze in the plan of the viewed displaying surface according to the position of the gaze determined in the eye reference mark.

7. The system according to claim 6, **characterized in that** the device (10) intended to support the mobile terminal comprises a second video camera (14) to provide the second video signal, the second video camera (14) being related to the support element (11) of the device (10) intended to support the mobile terminal and being ready to be directed towards the housing intended to receive the mobile terminal in order to capture images of viewed displaying surface, and **in that** the second video input interface (52) of the processing unit (50) is connected to a video output of the second video camera (14).

8. The system according to claim 6, **characterized in that** the second video input interface (52) of the processing unit (50) is connected to a video output of the mobile terminal ready to provide the second video signal.

9. The system according to any of claims 6 to 8, **characterized in that** the processing unit comprises a video output interface (54) ready to provide the second video signal wherein the images of the viewed displaying surface display a pointer representative of the gaze position within the plan of the viewed surface determined by the calculation module.

10. The system according to any of claims 6 to 9, **characterized in that** it comprises a module (60) for synchronizing the lighting of the first light source (L1) and the reference light sources (16a, 16b, 16c, 16d) with the capture of the first video signal by the first video camera (33) of the portable equipment, said synchronization module comprising an video input interface (61) ready to receive the first video signal of the first video camera (33) of the portable equipment, feeding means (63) ready to control, in a synchronous way, the switching on and off of the first light source and the reference light sources and means to process the received video signal arranged with said feeding means in order to control the lighting of the first light source and the reference light sources at the rate of one image of two relatively to the images captured by the first video camera of the portable equipment.

11. The system according to any of claims 6 to 10, **characterized in that** the support (30) of the portable equipment is a two branches spectacle frame, the arm (32) being fixed to one of the frame branches.

12. The system according to any of claims 6 to 11, **characterized in that** the arm (32) fitted on the support (30) of the portable equipment carries a second light source (L2) directed towards the eye near the lens of the first video camera (33) and intended to improve the eye illumination.

13. The system according to any of claims 6 to 12, **characterized in that** the plurality of reference light sources (16a, 16b, 16c, 16d) consists in four not aligned electroluminescent diodes when they are considered three by three.

14. The system according to any of claims 6 to 13, **characterized in that** the plate (12) of the device intended to support said mobile terminal is rotatably fitted to the support element (11) between at least two positions substantially at right angle therebetween, in order to respectively define a position known as portrait and a position known as landscape for the displaying surface of the mobile terminal intended to be received into the housing, and **in that** it comprises locking means (13) for the plate in each one of these respective positions.
